# EUROPEAN PATENT APPLICATION

(11) **EP 4 219 828 A1**
(43) Date of publication of application: **02.08.2023**
(21) Application number: 21902675.4
(22) Date of filing: 09.12.2021
(51) Int. Cl.: D21H 27/00, D21H 21/36, D21H 17/11, D21H 11/00, D21F 13/00, D21H 25/02, C12Q 1/18, G01N 1/28, C12R 1/19

(54) **METHOD FOR PREPARING BACTERIOSTATIC HOUSEHOLD PAPER**

(30) Priority: 10.12.2020 CN 202011454151
(71) Applicant: Zhejiang Jingxing Paper Joint Stock Co., Ltd., Pinghu, Zhejiang 314214 (CN)
(72) Inventor: CAO, Haibing, Town Pinghu, Zhejiang 314214 (CN); AN, Xingye, Town Pinghu, Zhejiang 314214 (CN); XU, Dong, Town Pinghu, Zhejiang 314214 (CN); LIU, Hongbin, Town Pinghu, Zhejiang 314214 (CN); ZHANG, Runqing, Town Pinghu, Zhejiang 314214 (CN); LU, Bin, Town Pinghu, Zhejiang 314214 (CN); DONG, Hongming, Town Pinghu, Zhejiang 314214 (CN)
(74) Representative: karo IP
(86) International application number: PCT/CN2021/136642
(87) International publication number: WO 2022/121970

(57) **Abstract**

The present invention relates to a method for preparing antibacterial household paper, sequentially including following steps: obtaining a paper sample, sterilizing the paper sample, thoroughly soaking in a benzalkonium bromide solution, disinfecting, obtaining a culture medium, making the paper sample adhere to the culture medium, and culturing; the advantages of the present invention are that in step 3, by soaking the paper sample thoroughly in the benzalkonium bromide solution to transfer the benzalkonium bromide solution, the paper sample may be thoroughly soaked with the solution, such that the benzalkonium bromide solution is adsorbed and distributed more uniformly on the paper sample; in step 7, the paper sample is cultured under 37°C, where the temperature 37°C provides a good growth environment for bacteria, such that the antibacterial effect of the paper sample can be noticeably identified at the end of culture. Therefore, the preparation method of the present invention is simple in operation, does not need to add additional expensive equipment and instruments, only needs to use a coating or applying device of a papermaking machine itself to load a bacteriostatic agent to spray on paper surfaces, and has a notable antibacterial effect.

## Description

### FIELD

The present invention relates to a method for preparing antibacterial household paper.

### BACKGROUND

Household paper, which is generally made from all-wood pulp and needs to be safe and hygienic, is paper for daily consumption and an indispensable sanitary article for home, travel, and catering. To enhance the antibacterial property of household paper, bamboo fiber household paper emerges; however, its antibacterial effect is not well recognized. The research and development of highly-effective antibacterial household paper will bring enormous economic and social benefits, such that it is particularly important to develop household paper products with a stronger antibacterial effect. The Chinese Patent Application No. 2020105640438 discloses an antibacterial facial tissue containing a natural bactericidal ingredient, and a preparing method and applications thereof, and specifically discloses an antibacterial composition prepared by mixing amino silicon oil, natural bactericidal active ingredient extract oil, nonionic emulsifier, glacial acetic acid, propylene glycol, and water, resulting in high consumption of the composition. In addition, the antibacterial facial tissue is prepared by spraying the antibacterial composition uniformly on the facial tissue, a consequence of which is that the antibacterial composition cannot infiltrate inside the paper, such that only the surface has an antibacterial property, rendering an instable antibacterial effect.

### SUMMARY

An objective of the present invention is to provide a method for preparing antibacterial household paper, which enhances antibacterial effect of the paper without utilizing excess chemicals.

To solve the above technical problem, the present invention is implemented by following technical solutions: a method for preparing antibacterial household paper, sequentially comprising:
step 1: obtaining a paper sample with a diameter of 1cm;
step 2: placing the obtained paper sample from the step 1 into a test tube for closed sterilization at a temperature of 120°C to 125°C for 20 min to 25 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 0% to 2%;
step 4: disinfecting the dried paper sample from the step 3 for 30 min to 45 min;
step 5: obtaining a culture medium coated with an *Escherichia coli* suspension;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

Preferably, the paper sample in the step 1 is prepared from a softwood pulp board, comprising:
511: weighing and taking the softwood pulp board and disintegrating it utill being free of interlaced fibers, then taking the disintegrated pulp out, spin-drying, and putting into a sealed bag, followed by putting the bag into a refrigerator, making moisture even, and then refrigerating the pulp for subsequent use;
S12: disintegrating the refrigerated pulp for subsequent use from the step S11 with a deflaker at 8000 r until fibers are completely disintegrated, and making a hand sheet of 40 g/m² to 60 g/m² quantitatively using a fast sheet former; and
S13: cutting the hand sheet from the step S12 into a paper sample with a diameter of 1 cm.

Preferably, a concentration of the benzalkonium bromide solution in the step 3 is 0%, or 0.13%, or 0.25%, or 0.5%, or 1%, or 1.5%, or 2%.

Preferably, the paper sample in the step 3 is placed into the benzalkonium bromide solution for thorough soak by sterilized tweezers.

Preferably, the *Escherichia coli* suspension in the step 5 contains 10² CFU/mL *Escherichia coli,* and the *Escherichia coli* suspension coated on the culture medium in the step 5 has a volume of 0.1 ml.

Preferably, the culture medium in the step 5 is placed in a culture dish with a thickness of 4 mm, the *Escherichia coli* suspension is placed into the culture dish, and the *Escherichia coli* suspension is uniformly coated on the culture medium by rotating the culture dish.

Preferably, the culture medium is a 2×YT culture medium, and the culture medium is sterilized before being placed into the culture dish.

Preferably, the paper sample in the step 4 is disinfected by ultraviolet light.

Preferably, the inhibition zone on the paper sample in the step 7 is measured by a ruler.

In view of the above, the present invention has the following advantages: in the present invention, the paper is prepared by the method including obtaining a paper sample, sterilizing the paper sample, thoroughly soaking in a benzalkonium bromide solution, disinfecting, obtaining a culture medium, making the paper sample adhere to the culture medium, and culturing in sequence; in the step 2, through the sterilization treatment of the paper sample, all bacterial spores may be killed to achieve complete sterilization; in the step 3, by soaking the paper sample thoroughly in the benzalkonium bromide solution to transfer the benzalkonium bromide solution, the paper sample can be thoroughly soaked with the solution, such that the benzalkonium bromide solution is adsorbed and distributed more uniformly on the paper sample, and by taking out the thoroughly soaked paper sample to remove an excess benzalkonium bromide solution, followed by drying, the excess benzalkonium bromide solution can be filtered out, and the drying ensures uniform dispersion of the benzalkonium bromide solution on the paper sample, reducing waste of the benzalkonium bromide solution; in the step 4, disinfecting the paper sample enables further removal of the bacteria carried in the paper sample, avoiding interference with the antibacterial result; in the step 5, the obtained culture medium provides the paper sample a culture environment for bacteria; in the step 6, adherence of the paper sample to the culture medium avoids the paper sample from detachment or displacement during bacterial culture. In the step 7, the paper sample is cultured under 37°C, where the temperature 37°C provides a good growth environment for bacteria, such that the antibacterial effect of the paper sample can be noticeably identified at the end of culture. Finally, the antibacterial effect of the paper sample is characterized by the inhibition zone on the paper sample, the size of the inhibition zone is conveniently measured, and the antibacterial degree of the paper sample can be obtained quickly and intuitively; in the step 3, the thorough soak with the benzalkonium bromide solution enables the benzalkonium bromide solution to be transferred onto the paper sample, the benzalkonium bromide solution is a common quaternary ammonium salt cationic surfactant broad-spectrum bactericide, which may inhibit bacteria at a low concentration, and benzalkonium bromide can reduce surface tension of the water solution, change cell membrane permeability, and denature the protein of bacteria, where the hydrophobic groups and hydrophilic groups in its molecular structure can penetrate into the lipid and protein layers of the cell plasma membrane, respectively, causing changes to the permeability of bacteria, and leading to I the loss of intracellular substances and the death of bacteria. Therefore, the preparation method of the present invention is simple in operation, does not need to add additional expensive equipment and instruments, only needs to use a coating or applying device of a papermaking machine itself to load a bacteriostatic agent to spray on paper surfaces, and has a notable antibacterial effect.

### DETAILED DESCRIPTION

### Embodiment 1

A method for preparing antibacterial household paper sequentially includes:
step 1: obtaining a paper sample with a diameter of 1 cm;
step 2: placing the obtained paper sample from the step 1 into a test tube for closed sterilization a temperature of 121°C for 20 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 0%;
step 4: disinfecting the dried paper sample from the step 3 by ultraviolet light for 30 min;
step 5: placing a sterilized culture medium into a culture dish with a thickness of 4 mm, followed by placing an *Escherichia coli* suspension into the culture dish, and rotating the culture dish to uniformly coat the *Escherichia coli* suspension onto the culture medium to obtain a culture medium coated with the *Escherichia coli* suspension, where the *Escherichia coli* suspension contains 10² CFU/mL *Escherichia coli,* a volume of the *Escherichia coli* suspension is 0.1 ml, and the culture medium is a 2×YT culture medium;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

### Embodiment 2

A method for preparing antibacterial household paper sequentially includes:
step 1: obtaining a paper sample with a diameter of 1 cm;
step 2: placing the obtained paper sample from the step 1 into a test tube for closed sterilization at a temperature of 120°C for 25 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 0.13%;
step 4: disinfecting the dried paper sample from the step 3 by ultraviolet light for 35 min;
step 5: placing a sterilized culture medium into a culture dish with a thickness of 4 mm, followed by placing an *Escherichia coli* suspension into the culture dish, and rotating the culture dish to uniformly coat the *Escherichia coli* suspension onto the culture medium to obtain a culture medium coated with the *Escherichia coli* suspension, where the *Escherichia coli* suspension contains 10² CFU/mL *Escherichia coli,* a volume of the *Escherichia coli* suspension is 0.1 ml, and the culture medium is a 2×YT culture medium;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

### Embodiment 3

A method for preparing antibacterial household paper sequentially includes:
step 1: obtaining a paper sample with a diameter of 1 cm;
step 2: placing the obtained paper sample from the step 1 into a test tube for closed sterilization at a temperature of 122°C for 23 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 0.25%;
step 4: disinfecting the dried paper sample from the step 3 by ultraviolet light for 35 min;
step 5: placing a sterilized culture medium into a culture dish with a thickness of 4 mm, followed by placing an *Escherichia coli* suspension into the culture dish, and rotating the culture dish to uniformly coat the *Escherichia coli* suspension onto the culture medium to obtain a culture medium coated with the *Escherichia coli* suspension, where the *Escherichia coli* suspension contains 10² CFU/mL *Escherichia coli,* a volume of the *Escherichia coli* suspension is 0.1 ml, and the culture medium is a 2×YT culture medium;
step 6: placing the disinfected paper sample from the step 4 into the obtained culture medium resulting from the step 5 to make it fully adhere to the culture medium; and
step 7: culturing the treated paper sample from the step 6 under 37°C, and measuring an inhibition zone on the paper sample to characterize an antibacterial effect of the paper sample.

### Embodiment 4

A method for preparing antibacterial household paper sequentially includes:
step 1: obtaining a paper sample with a diameter of 1 cm;
step 2: placing the obtained paper sample from the step 1 into a test tube for closed sterilization at a temperature of 120°C for 22 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 0.5%;
step 4: disinfecting the dried paper sample from the step 3 by ultraviolet light for 32 min;
step 5: placing a sterilized culture medium into a culture dish with a thickness of 4 mm, followed by placing an *Escherichia coli* suspension into the culture dish, and rotating the culture dish to uniformly coat the *Escherichia coli* suspension onto the culture medium to obtain a culture medium coated with the *Escherichia coli* suspension, where the *Escherichia coli* suspension contains 10² CFU/mL *Escherichia coli,* a volume of the *Escherichia coli* suspension is 0.1 ml, and the culture medium is a 2×YT culture medium;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

### Embodiment 5

A method for preparing antibacterial household paper sequentially includes:
step 1: obtaining a paper sample with a diameter of 1 cm;
step 2: placing the obtained paper sample from the step 1 into a test tube for closed sterilization at a temperature of 121°C for 21 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 1%;
step 4: disinfecting the dried paper sample from the step 3 by ultraviolet light for 31 min;
step 5: placing a sterilized culture medium into a culture dish with a thickness of 4 mm, followed by placing an *Escherichia coli* suspension into the culture dish, and rotating the culture dish to uniformly coat the *Escherichia coli* suspension onto the culture medium to obtain a culture medium coated with the *Escherichia coli* suspension, where the *Escherichia coli* suspension contains 10² CFU/mL *Escherichia coli,* a volume of the *Escherichia coli* suspension is 0.1 ml, and the culture medium is a 2×YT culture medium;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

### Embodiment 6

A method for preparing antibacterial household paper sequentially includes:
step 1: obtaining a paper sample with a diameter of 1 cm;
step 2: placing the obtained paper sample from the step 1 into a test tube for closed sterilization at a temperature of 123°C for 21 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 1.5%;
step 4: disinfecting the dried paper sample from the step 3 by ultraviolet light for 35 min;
step 5: placing a sterilized culture medium into a culture dish with a thickness of 4 mm, followed by placing an *Escherichia coli* suspension into the culture dish, and rotating the culture dish to uniformly coat the *Escherichia coli* suspension onto the culture medium to obtain a culture medium coated with the *Escherichia coli* suspension, where the *Escherichia coli* suspension contains 10² CFU/mL *Escherichia coli,* a volume of the *Escherichia coli* suspension is 0.1 ml, and the culture medium is a 2×YT culture medium;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

### Embodiment 7

A method for preparing antibacterial household paper sequentially includes:
step 1: obtaining a paper sample with a diameter of 1 cm;
step 2: placing the obtained paper sample from the step 1 into a test tube for closed sterilization at a temperature of 124°C for 24 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 2%;
step 4: disinfecting the dried paper sample from the step 3 by ultraviolet light for 35 min;
step 5: placing a sterilized culture medium into a culture dish with a thickness of 4 mm, followed by placing an *Escherichia coli* suspension into the culture dish, and rotating the culture dish to uniformly coat the *Escherichia coli* suspension onto the culture medium to obtain a culture medium coated with the *Escherichia coli* suspension, where the *Escherichia coli* suspension contains 10² CFU/mL *Escherichia coli,* a volume of the *Escherichia coli* suspension is 0.1 ml, and the culture medium is a 2×YT culture medium;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

The inhibition zones on the paper sample from Embodiment 1, Embodiment 2, Embodiment 3, Embodiment 4, Embodiment 5, Embodiment 6, and Embodiment 7 are measured, respectively. Specific measurement results are shown in Table 1:

**Table 1**

| | | | | | | | |
|---|---|---|---|---|---|---|---|
| Benzalkonium bromide solution (%) | 0 | 0.13 | 0.25 | 0.5 | 1 | 1.5 | 2 |
| Inhibition zone diameter (mm) | 10 | 11.35 | 12.77 | 14.93 | 18.44 | 19.5 | 23.76 |

In view of the above, the present invention has the following advantages: in the present invention, the paper is prepared by the method including obtaining a paper sample, sterilizing the paper sample, thoroughly soaking in a benzalkonium bromide solution, disinfecting, obtaining a culture medium, making the paper sample adhere to the culture medium, and culturing in sequence; in the step 2, through the sterilization treatment of the paper sample, all bacterial spores can be killed to achieve complete sterilization; in the step 3, by soaking the paper sample thoroughly in the benzalkonium bromide solution to transfer the benzalkonium bromide solution, the paper sample can be thoroughly soaked with the solution, such that the benzalkonium bromide solution is adsorbed and distributed more uniformly on the paper, and by taking out the thoroughly soaked paper sample to remove an excess benzalkonium bromide solution, followed by drying, the excess benzalkonium bromide solution can be filtered out, and the drying ensures uniform dispersion of the benzalkonium bromide solution on the paper sample, reducing waste of the benzalkonium bromide solution; in the step 4, disinfecting the paper sample enables further removal of the bacteria carried in the paper sample, avoiding interference with the antibacterial result; in the step 5, the obtained culture medium provides the paper sample a culture environment for bacteria; in the step 6, adherence of the paper sample to the culture medium avoids the paper sample from detachment or displacement during bacterial culture. In the step 7, the paper sample is cultured under 37°C, where the temperature 37°C provides a good growth environment for bacteria, such that the antibacterial effect of the paper sample can be noticeably identified at the end of culture. Finally, the antibacterial effect of the paper sample is characterized by the inhibition zone on the paper sample, the size of the inhibition zone is conveniently measured, and the antibacterial degree of the paper sample can be obtained quickly and intuitively; in the step 3, the thorough soak with the benzalkonium bromide solution enables the benzalkonium bromide solution to be transferred onto the paper sample, the benzalkonium bromide solution is a common quaternary ammonium salt cationic surfactant broad-spectrum bactericide, which may inhibit bacteria at a low concentration, and benzalkonium bromide can reduce surface tension of the water solution, change cell membrane permeability, and denature the protein of bacteria, where the hydrophobic groups and hydrophilic groups in its molecular structure can penetrate into the lipid and protein layers of the cell plasma membrane, respectively, causing changes to the permeability of bacteria, and leading to I the loss of intracellular substances and the death of bacteria. Therefore, the preparation method of the present invention is simple in operation, does not need to add additional expensive equipment and instruments, only needs to use a coating or applying device of a papermaking machine itself to load a bacteriostatic agent to spray on paper surfaces, and has a notable antibacterial effect. °C°C

In addition to the above preferred embodiments, the present invention also has other implementations. Those skilled in the art may make various alterations and modifications according to the present invention, and all such alterations and modifications fall within the scope defined by the appended claims without departing from the spirits of the present invention.

## Claims

1. A method for preparing antibacterial household paper, **characterized by** sequentially comprising:
step 1: obtaining a paper sample with a diameter of 1 cm;
step 2: placing the obtained paper sample from the step 1 into a test tube for closed sterilization at a temperature of 120°C to 125°C for 20 min to 25 min;
step 3: placing the sterilized paper sample from the step 2 into a benzalkonium bromide solution for thorough soak, and taking out the thoroughly soaked paper sample to remove an excess benzalkonium bromide solution, followed by drying, and a concentration of the benzalkonium bromide solution is 0% to 2%;
step 4: disinfecting the dried paper sample from the step 3 for 30 min to 45 min;
step 5: obtaining a culture medium coated with an *Escherichia coli* suspension;
step 6: placing the disinfected paper sample from the step 4 into the obtained culture medium from the step 5 to make it fully adhere to the culture medium; and
step 7: culturing the treated paper sample from the step 6 under 37°C, and measuring an inhibition zone on the paper sample to characterize an antibacterial effect of the paper sample.

2. The method for preparing antibacterial household paper according to claim 1, **characterized in that** the paper sample in the step 1 is prepared from a softwood pulp board, comprising:
S11: weighing and taking the softwood pulp board and disintegrating it until being free of interlaced fibers, then taking the disintegrated pulp out, spin-drying, and putting into a sealed bag, followed by putting the bag into a refrigerator, making moisture even, and then refrigerating the pulp for subsequent use;
S12: disintegrating the refrigerated pulp for subsequent use from the step S11 with a deflaker at 8000 r until fibers are completely disintegrated, and making a hand sheet of 40 g/m² to 60 g/m² quantitatively with a fast sheet former; and
S13: cutting the hand sheet from the step S12 into a paper sample with a diameter of 1 cm.

3. The method for preparing antibacterial household paper according to claim 1, **characterized in that** a concentration of the benzalkonium bromide solution in the step 3 is 0%, or 0.13%, or 0.25%, or 0.5%, or 1%, or 1.5%, or 2%.

4. The method for preparing antibacterial household paper according to claim 1, **characterized in that** the paper sample in the step 3 is placed into the benzalkonium bromide solution for thorough soak by sterilized tweezers.

5. The method for preparing antibacterial household paper according to claim 1, **characterized in that** the *Escherichia coli* suspension in the step 5 contains 10² CFU/mL *Escherichia coli,* and the *Escherichia coli* suspension coated on the culture medium in the step 5 has a volume of 0.1 ml.

6. The method for preparing antibacterial household paper according to claim 5, **characterized in that** the culture medium in the step 5 is placed in a culture dish with a thickness of 4 mm, the *Escherichia coli* suspension is placed into the culture dish, and the *Escherichia coli* suspension is uniformly coated on the culture medium by rotating the culture dish.

7. The method for preparing antibacterial household paper according to claim 6, **characterized in that** the culture medium is a 2×YT culture medium, and the culture medium is sterilized before being placed into the culture dish.

8. The method for preparing antibacterial household paper according to claim 1, **characterized in that** the paper sample in the step 4 is disinfected by ultraviolet light.

9. The method for preparing antibacterial household paper according to claim 1, **characterized in that** the inhibition zone on the paper sample in the step 7 is measured by a ruler.
